# EUROPEAN PATENT SPECIFICATION

(11) **EP 2 224 866 B1**
(45) Date of publication and mention of the grant of the patent: **10.05.2017**
(21) Application number: 08840153.4
(22) Date of filing: 14.10.2008
(51) Int. Cl.: A61B 17/70

(54) **FLEXIBLE MEMBER WITH VARIABLE FLEXIBIILITY FOR PROVIDING DYNAMIC STABILITY TO A SPINE**
FLEXIBLES ELEMENT MIT VARIABLER FLEXIBILITÄT, UM EINER WIRBELSÄULE DYNAMISCHE STABILITÄT ZU VERLEIHEN
ÉLÉMENT FLEXIBLE DE FLEXIBILITÉ VARIABLE POUR CONFÉRER UNE STABILITÉ DYNAMIQUE À UNE COLONNE VERTÉBRALE

(30) Priority: 16.10.2007 US 873102
(43) Date of publication of application: 08.09.2010
(73) Proprietor: Zimmer Spine, Inc., Minneapolis, MN 55439 (US)
(72) Inventor: HESTAD, Hugh D., Edina, MN 55410 (US); OTTE, John F., St. Anthony, MN 55418 (US); HILLYARD, Angela L., Greenfield, MN 55373 (US); BOSCHERT, Paul F., Minneapolis, MN 55406 (US); DARST RICE, Mark W., Minneapolis, MN 55417 (US); LANCIAL, Mike E., Minneapolis, MN 55426 (US)
(74) Representative: Manitz Finsterwald Patentanwälte PartmbB
(86) International application number: PCT/US2008/079872
(87) International publication number: WO 2009/052115

(56) References cited:
- EP-A1- 1 800 613
- WO-A-2004/089244
- WO-A-2007/044795
- WO-A-2007/087476
- US-A1- 2006 111 715
- US-A1- 2007 225 710

## Description

### Field of the Invention

The present invention relates generally to spinal support devices and, more specifically, to a flexible member having variable flexibility for use with a dynamic stabilization system to provide dynamic stability to a person's spine.

### Background of the Invention

The treatment of acute and chronic spinal instabilities or deformities of the thoracic, lumbar, and sacral spine has traditionally involved the implantation of rigid rods to secure the vertebrae of a patient. More recently, flexible materials have been utilized in connection with anchor members, e.g., pedicle screws, to provide a dynamic stabilization of the spinal column. Such dynamic stabilization systems or implants typically include a flexible member positioned between pedicle screws installed in adjacent vertebrae of a person's spine.

Certain dynamic stabilization systems permit the top loading of a flexible member and connecting member between pedicle screws. One such top loading system is disclosed in U.S. Patent Application Publication No. 2002/0035366 to Walder et al., titled "Pedicle Screw For Intervertebral Support Elements". Another top loading system is disclosed in U.S. Patent Application Serial No. 11/618,943 to Hestad et al., titled "Spine Stiffening Device". Still other dynamic stabilization systems are adapted to securely retain the flexible member between pedicle screws without the use of a connecting member.

While current dynamic stabilization systems include flexible members, these flexible members typically are of a uniform cylindrical shape, which may not allow for variability in flexibility, except by varying the length of the flexible member between pedicle screws. In an effort to modify the flexibility of the flexible member at one or more locations along its length, some flexible members are being composed of more than one material, which have different degrees of flexibility. However, the processes for manufacturing the multi-material flexible members and the additional material itself can be cost prohibitive. In addition, while some single material flexible members are known to provide variations from the typical cylindrical configuration, e.g., a spiral-patterned flexible member, additional configurations, such as nonuniform or atypical configurations, are needed for providing flexible members with other desirable bending movements. Indeed, other atypical configurations would be beneficial for providing surgeons with greater options in selecting the most appropriate flexible member for placement at a specific location along a patient's spine, such selection being dictated by the desired bending movement of the flexible member at that location. US 2006/0111715 A1 discloses a prior art spine stabilization system in accordance with the preamble of claim 1. Further prior art systems are disclosed in the following documents EP 1 800 613 A1, WO 2007/044795, WO 2004/089244, US 2007/225710 and WO 2007/087476.

Accordingly, it would be desirable to provide flexible members having variable flexibility attributable to a specified configuration for use with dynamic stabilization systems to provide dynamic stability to a person's spine that addresses the above and other deficiencies of current flexible members.

### Summary of the Invention

The above object is satisfied by a spine stabilization system in accordance with claim 1, by a spine stabilization system in accordance with claim 13 and by a spine stabilization system in accordance with claim 15.

Further embodiments of the invention are defined in the dependent claims.

These and other various configurations of the flexible member can allow for a desired bending of the flexible member, such as easier bending in one direction relative to another, as compared to conventional or typical flexible members which have equal bending force in all directions.

One or more flexible members can be utilized in a method for treating the spine of a patient.

The features and objectives of the present invention will become more readily apparent from the following Detailed Description taken in conjunction with the accompanying drawings.

### Brief Description of the Drawing

The accompanying drawings, which are incorporated in and constitute a part of this specification, illustrate an embodiment of the invention and, together with the general description of the invention given above, and detailed description given below, serve to explain the invention.
FIG. 1A is a side elevational view of a dynamic stabilization system including anchor members inserted into the spinal column and a flexible member secured therebetween;
FIG. 1B is a side elevational view of a dynamic stabilization system including top loading anchor members inserted into the spinal column and a flexible member with connecting member being secured therebetween;
Fig. 2A is a perspective view of the flexible member of Fig. 1A;
Fig. 2B is a perspective view of the flexible member of Fig. 1B;
Fig. 3 is a cross-sectional view of the flexible member of Fig. 2B taken along the line 3-3;
Fig. 4 is a perspective view of another embodiment of a flexible member;
Fig. 4A is a cross-sectional view of the flexible member of Fig. 4 taken along the line 4A-4A;
Fig. 5 is a perspective view of another embodiment of a flexible member;
Fig. 5A is a cross-sectional view of the flexible member of Fig. 5 taken along the line 5A-5A;
Fig. 6 is a perspective view of another embodiment of a flexible member;
Fig. 6A is a cross-sectional view of the flexible member of Fig. 6 taken along the line 6A-6A;
Figs. 7-16 are perspective views of various embodiments of a flexible member;
Fig. 17A is a disassembled, perspective view of an embodiment of a connecting member with the flexible member of Fig. 1B for use in a dynamic stabilization system;
Fig. 17B is a partially disassembled view of a dynamic stabilization system utilizing the connecting member and flexible member shown in Fig. 17A and top loading anchor members; and
Fig. 17C is a cross-sectional view of the assembled dynamic stabilization system of Fig. 17B.

### Detailed Description of the Invention

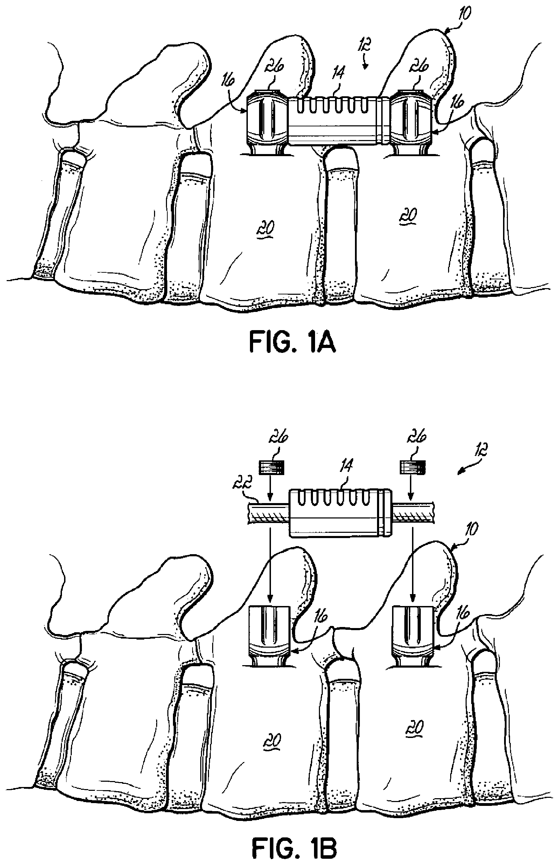

Figs. 1A and 1B illustrate cut-away sections of a spine 10 having a dynamic stabilization system or implant 12 implanted therein. The systems 12 of Figs. 1A and 1B, include a flexible member 14 having variable flexibility positioned between anchor members 16, for example, pedicle screws, installed in adjacent vertebrae 20 of the spine 10.

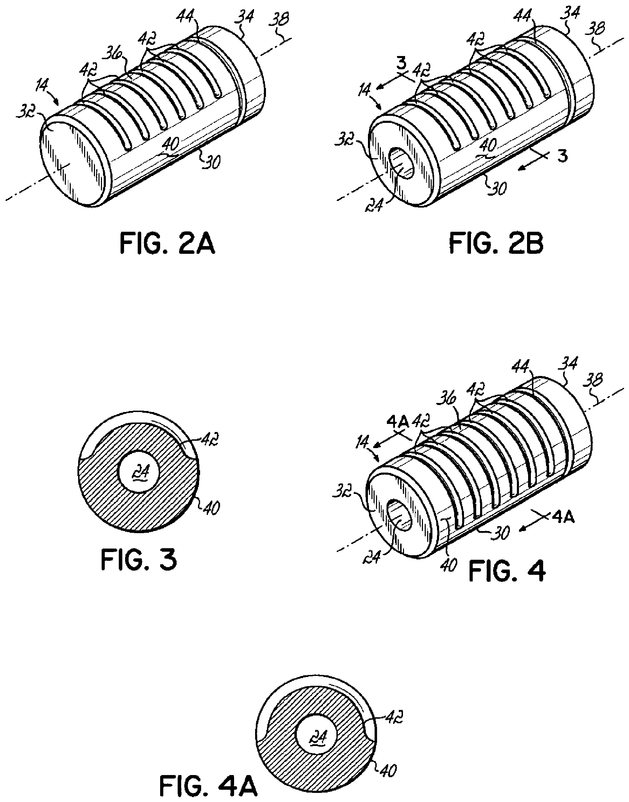

The anchor members 16 of Figs. 1A and 1B generally illustrate top loading pedicle screws that retain the flexible members 14 therebetween by means well known in the art. One such top loading type screw is disclosed in U.S. Patent Application Publication No. 2002/0035366 to Walder et al., titled "Pedicle Screw For Intervertebral Support Elements". With further reference to Fig. 1B, a connecting member 22 may be passed through an aperture 24 (Fig. 2B) in the flexible member 14, such connecting member 22 then being top loaded and secured within a top portion of each anchor member 16 by threadable cap members 26. The connecting member 22 can be passed through the aperture 24 during or prior to implantation in a patient, or preformed or coupled to the flexible member 14 to form a unitary structure during manufacture of the dynamic stabilization system 12. Once secured, that connecting member 22 retains the flexible member 14 between the anchor members 16 while cooperating with the flexible member 14 for permitting mobility of the spine 10. In contrast, the flexible member 14 of Fig. 1A is devoid of aperture 24 and corresponding connecting member 22 and, instead, is directly top loaded into anchor members 16 and securely held in place by threadable cap members 26.

The connecting member 22 may generally include a flexible structure made from materials such as NiTiNOL, a stainless steel coiled wire, or a polymer-based material like polyethylene-terephthalate. Alternatively, the connecting member 22 can be a rigid structure or a combination of a rigid and flexible structure for connection to anchor members 16. It will be recognized that various other materials suitable for implantation of the connecting member 22 within the human body and for providing stabilization of the spine while maintaining flexibility may be used.

In accordance with embodiments of the present invention, the flexible members 14 of Figs. 1A and 1B, as best shown in Figs. 2A and 2B, respectively, include a body 30 including opposing first and second ends 32 and 34 connected by an intermediate portion 36 extending therebetween. In this embodiment, each opposing end 32, 34 is configured for cooperation with a corresponding anchor member 16 and the body 30 has a cylindrical shape. The body 30 further includes a lengthwise central axis 38 and an outer surface 40 defining a circumference and having a plurality of spaced-apart grooves 42 therein situated perpendicular to the lengthwise axis 38 of the body 30. As best shown in Fig. 3, the grooves 42 extend around no more than half the circumference of the body 30. Optional groove 44 is further situated perpendicular to the lengthwise axis 38 of the body 30 proximate the second end 34, and extends around the full circumference of the body 30. The grooves 42, 44 provide the flexible member 14 with a variable flexibility as discussed further below. The flexible member 14 of Fig. 2B further includes aperture 24 extending lengthwise through the body 30 for receiving the connecting member 22.

While six grooves 42 are shown in Figs. 2A and 2B, it should be understood that more or less than six grooves 42 may be provided. Also, the spacing between grooves 42 may be equal, unequal, or a mixture thereof as desired. And, although grooves 42 are shown as being perpendicular to the lengthwise axis 38, one or more grooves 42 may be slightly askew or substantially perpendicular thereto. Furthermore, even though the grooves 42, 44 are shown, for example, as extending around less than half the circumference or around the entire circumference, respectively, variations of the length thereof are readily understood. By way of example, Figs. 4 and 4A show a flexible member 14 similar to Fig. 2B that has a plurality of spaced-apart grooves 42 situated perpendicular to the lengthwise axis 38 of the body 30 and optional groove 44. However, the grooves 42, as best shown in Fig. 4A, extend around approximately half the circumference of the body.

Additionally, despite the absence of a connecting member 22 in the system 12 of Fig. 1A, it should be understood by one of ordinary skill in the art that the flexible member 14 of Fig. 2B could be used with the system 12 of Fig. 1A. The flexible member 14 also may be provided in varying lengths, e.g., twelve-inch lengths, so that a surgeon can cut, or shape, the flexible member 14 to fit between opposing anchor members 16 along a specific section of spine 10, as well as to accommodate a desired bending movement of the flexible member 14. In addition, the optional groove 44, which is situated near the second end 34, can allow the surgeon to securely grip, or hold, the flexible member 14, for example, with a tool (not shown), such as a clamp, so that the flexible member 14 can be cut to a desired size. It should be understood that the optional groove 44 may be provided near the first end 32 or at both the first and second ends 32, 34.

Orientation of the flexible member 14, e.g., inferior or superior positioning of one end 32, 34 relative to the spine 10 and/or lateral versus anterior/posterior positioning of grooves 42 is determined by the desired bending movement of the selected flexible member 14 at that specific section of spine 10. In other words, orientation of the flexible member 14 is generally determined based upon the needs of the patient, with the flexible member 14 of the present invention allowing for tailoring thereof on a patient-by-patient basis. In addition, although the flexible member 14 is illustrated as being cylindrical, it should be understood by one having ordinary skill in the art that other desired shapes, for example, square, rectangular, oval, etc. may be utilized.

With respect to the bending movement of the flexible member 14, the size, i.e., depth, width, and length, of the groove 42, 44 as well as the number thereof generally determine the degree and variability of flexibility for the flexible member 14. For example, the flexible member 14 will both flex and extend more easily at the location of optional groove 44, which again extends around the full circumference, as compared to areas devoid of such groove 44. And, with respect to grooves 42, both individually and collectively, the flexible member 14, when the ends 32, 34 are forced in a direction toward grooves 42, will flex more easily as compared to areas that are devoid of such grooves 42. In contrast, when the ends 32, 34 are forced in a direction away from grooves 42, the flexible member 14 may not experience the same ease of flexibility. Such differential in flexion as compared to extension may be generally attributed to the grooves 42 extending around no more than half the circumference. Therefore, if grooves 42 of the flexible member 14 are located anterior relative to the spine 10, the flexible member 14 can allow for easier bending anteriorly as compared to posteriorly or laterally. Consequently, the flexible member 14 could be rotated 180 degrees, for example, and then the anterior and lateral bending would require more force to allow similar ease of bending in contrast to posterior bending.

The surgeon implanting the dynamic stabilization system 12 can selectively take advantage of the varying flexibility of flexible member 14 to treat an indication or condition in the patient. The surgeon can be provided with a plurality of pre-constructed systems 12 that have flexible members 14 with varying flexibility characteristics, or, alternatively, be provided with a variety of flexible members 14 with varying flexibility characteristics any one of which can be incorporated into a system 12 that is constructed during the surgical procedure.

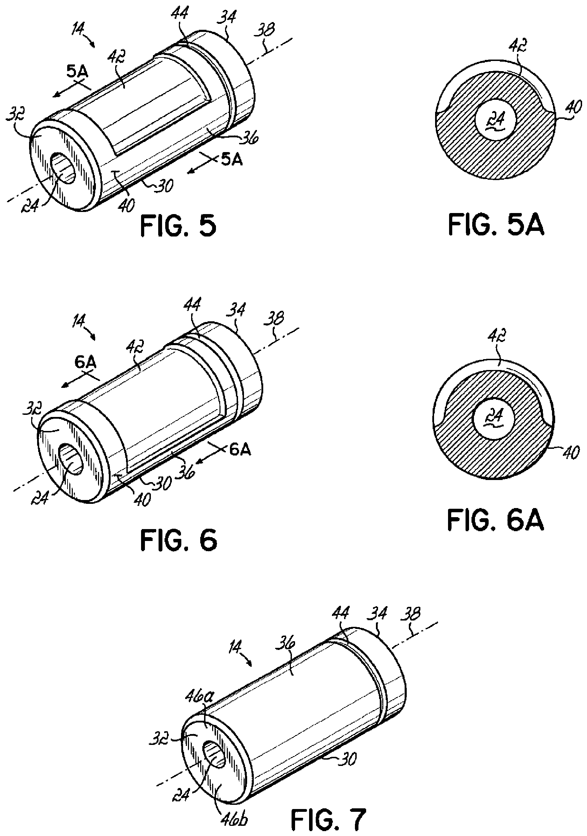

Fig. 5 depicts another embodiment of flexible member 14, which includes the body 30 of a cylindrical shape including opposing first and second ends 32, 34 connected by the intermediate portion 36 extending therebetween. The body 30 includes a lengthwise central axis 38, and an outer surface 40 defining a circumference and having groove 42 therein of a width greater than half the length of the body 30. The groove 42 further is situated perpendicular to the lengthwise axis 38 of the body 30 and, as best shown in Fig. 5A, extends around no more than half the circumference of the body 30. Optional groove 44 is also situated perpendicular to the lengthwise axis 38 of the body 30 proximate the second end 34, and extends around the full circumference of the body 30. The flexible member 14 further includes optional aperture 24 extending lengthwise through the body 30 for receiving connecting member 22.

Figs. 6 and 6A show a flexible member 14 similar to Figs. 5 and 5B, respectively, that has groove 42 of a width greater than half the length of the body 30 and optional groove 44. However, groove 42, as best shown in Fig. 6A, extends around approximately half the circumference of the body 30. The flexible member 14 shown in Figs. 5 and 6 provides a cross-sectional and lengthwise variability in flexibility that is dependent upon its configuration.

Fig. 7 depicts another embodiment of flexible member 14, which includes the cylindrical body 30 including opposing first and second ends 32, 34 connected by the intermediate portion 36 extending therebetween. The body 30 includes lengthwise central axis 38 and an outer surface 40 defining a circumference. Groove 44 is further situated perpendicular to the lengthwise axis 38 of the body 30 proximate the second end 34, and extends around the full circumference of the body 30. The flexible member 14 further includes optional aperture 24 extending lengthwise through the body 30 for receiving connecting member 22. Aperture 24 is positioned offset from the lengthwise axis 38. This offset positioning affords the flexible member 14 with opposing lengthwise areas 46a and 46b, which are disposed about the aperture 24, that differ in thicknesses and, thus, provide the flexible member 14 with variable flexibility. It should be understood that the thinnest area 46a is the most flexible with the thickest area 46b being the least flexible.

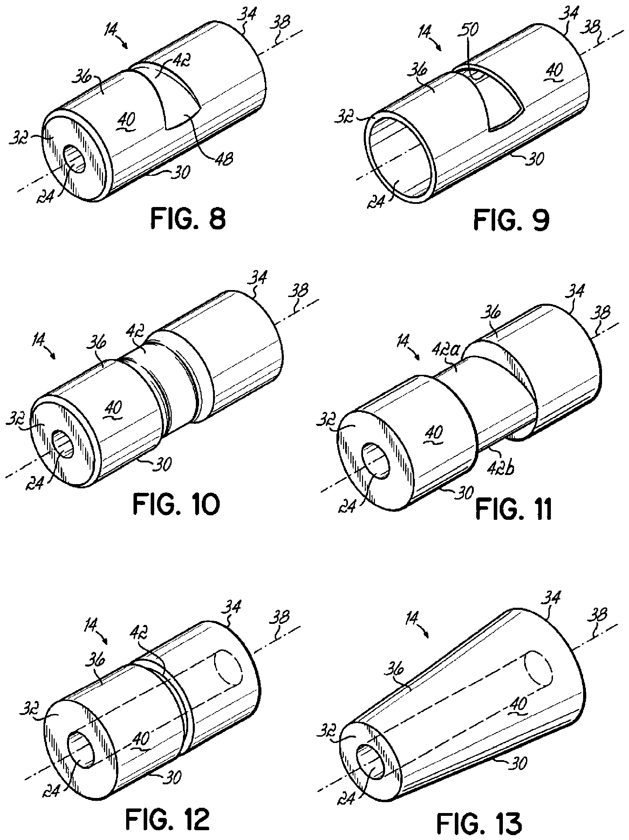

Fig. 8 depicts another embodiment of flexible member 14, which includes cylindrical body 30 including opposing first and second ends 32, 34 connected by intermediate portion 36 extending therebetween. The body 30 includes lengthwise central axis 38, and an outer surface 40 defining a circumference and having a groove 42 therein with opposing flared ends 48 (only one shown). The groove 42 is situated perpendicular to the lengthwise axis 38 of the body 30 and extends around no more than half the circumference. Although the groove 42 is shown substantially directly in-between the ends 32, 34, it should be understood that it could be provided closer to either of the first or second ends 32, 34 as desired. The flexible member 14 further includes optional aperture 24 extending lengthwise through the body 30 for receiving connecting member 22.

Fig 9 depicts another embodiment of flexible member 14, which is similar to Fig. 8, except that body 30 is tubular-shaped. In other words, the diameter of optional aperture 24 of Fig. 8 is greatly enlarged. In addition, the groove 42 of Fig. 8 now cooperates with aperture 24 to define, as shown in Fig. 9, an opening 50 in body 30.

Fig. 10 depicts yet another embodiment of flexible member 14, which includes cylindrical body 30 including opposing first and second ends 32, 34 connected by intermediate portion 36 extending therebetween. The body 30 includes lengthwise central axis 38, and an outer surface 40 defining a circumference and having groove 42 therein substantially directly in-between the ends 32, 34. The groove 42 is situated perpendicular to the lengthwise axis 38 of the body 30, extends around the full circumference, and has a width greater than about one-third and less than about two-thirds, e.g., about one half, the full length of the body 30 such that the body 30 substantially defines a dumbbell shape. Although, the groove 42 is shown directly in-between the ends 32, 34, it should be understood that it could be provided closer to either of the first or second ends 32, 34 as desired. The depicted configuration allows the ends 32, 34 to move, e.g., flex, generally independently of one another. The flexible member 14 further includes optional aperture 24 extending lengthwise through the body 30 for receiving the connecting member 22.

Fig. 11 depicts yet another embodiment of flexible member 14, which includes cylindrical body 30 including opposing first and second ends 32, 34 connected by intermediate portion 36 extending therebetween. The body 30 includes lengthwise central axis 38 and an outer surface 40 defining a circumference. The body 30 further includes a pair of grooves 42a and 42b situated in opposing relation with each extending around less than half the circumference of the body 30. Each groove 42a, 42b increases in depth in a direction from opposing ends to a center of the groove 42a, 42b to define crescent-shaped grooves. Such grooves 42a, 42b, are situated substantially directly in-between the ends 32, 34 with the intermediate portion 36 being substantially oval-shaped when viewed in cross-section perpendicular to the lengthwise axis 38 of the body 30. This configuration, similar to Fig. 10, allows the ends 32, 34 to move generally independently of one another with the exception that the flexible member 14 does not yield an equal bending force in all directions collectively about grooves 42a, 42b. The flexible member 14 further includes optional aperture 24 extending lengthwise through the body 30 for receiving the connecting member 22.

Fig. 12 depicts yet another embodiment of flexible member 14, which is similar to Fig. 10. However, groove 42 is much smaller in width as compared to groove 42 of Fig. 10, which has a width greater than about one-third and less than about two-thirds the full length of body 30. This smaller width limits the range of motion of the flexible member 14 about the groove 42.

Fig. 13 depicts another embodiment of flexible member 14, which includes cylindrical body 30 including opposing first and second ends 32, 34 connected by intermediate portion 36 extending therebetween. The body 30 includes lengthwise central axis 38 and optional aperture 24 extending lengthwise through the body 30 for receiving the connecting member 22. The aperture 24 maintains a constant diameter while the flexible member 14 includes a taper in the diameter of the body 30 from the second end 34 to the first end 32 to provide the flexible member 14 with a variable flexibility. Specifically, with the tapered configuration, the body 30 decreases in thickness from the second end 34 towards the first end 32 thereby defining a flexibility gradient along its length. It should be understood that the thinnest area, i.e., the first end 32, is the most flexible area with the thickest area, i.e., the second end 34, being the least flexible.

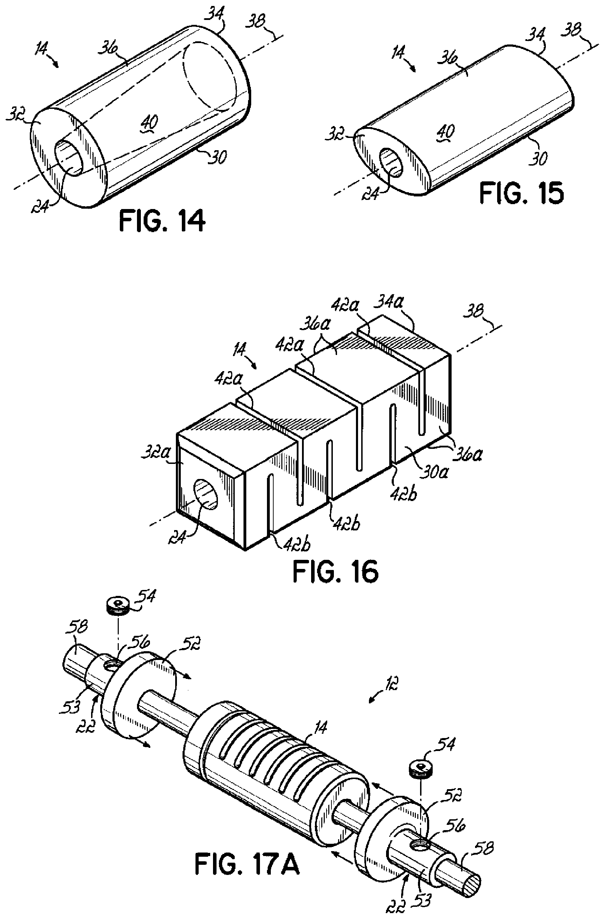

Fig. 14 depicts another embodiment of flexible member 14, which is a variation of the embodiment depicted in Fig. 13. Rather than including a taper in diameter of the body 30, the flexible member 14 includes a taper in the diameter of the aperture 24 as it extends lengthwise through the body 30 from the second end 34 to the first end 32. The cylindrical body 30 maintains a constant diameter. With this tapered configuration, the body 30 decreases in thickness from the first end 32 towards the second end 34 similarly defining a flexibility gradient along its length to provide the flexible member 14 with a variable flexibility.

Fig. 15 depicts another embodiment of flexible member 14, which includes cylindrical body 30 including opposing first and second ends 32, 34 connected by intermediate portion 36 extending therebetween. The body 30 is substantially oval-shaped along its length when viewed from both ends 32, 34 to provide the flexible member 14 with a variable flexibility. The body 30 further includes lengthwise central axis 38 and optional aperture 24 extending lengthwise through the body 30 for receiving the connecting member 22. The flexible member 14 shown in Fig. 15 does not yield an equal bending force along its length in all directions but rather provides variable flexibility, which is dependent upon its oval-shaped configuration.

Fig. 16 depicts yet another embodiment of flexible member 14, which includes body 30a defining a rectangular prism that includes opposing first and second rectangular bases 32a, 34a connected by four rectangular lateral faces 36a extending therebetween. The body 30a further includes lengthwise central axis 38 and first plurality of grooves 42a and a second plurality of grooves 42b. The grooves 42a, 42b are spaced offset from one another and situated in and along the full width of opposing lateral faces 36a of the body 30a perpendicular to the lengthwise axis 38 such that the body 30a is substantially serpentine-shaped to provide the flexible member 14 with a variable flexibility. The body 30a further includes optional aperture 24 extending lengthwise through the body 30a for receiving the connecting member 22.

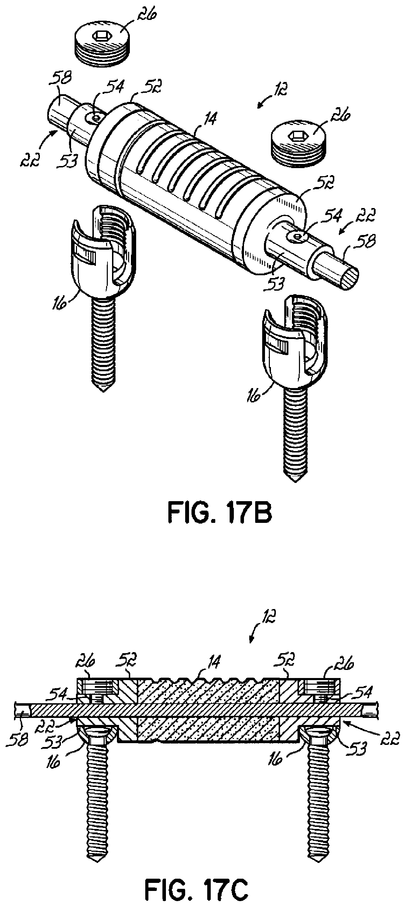

Referring now to Figs. 17A-17C, an alternative embodiment of dynamic stabilization system or implant 12 is shown including flexible member 14 of Fig. 1B positioned between anchor members 16. In this embodiment, the connecting members 22 include flanges 52 provided with outwardly projecting annular hubs 53 and a securing element in the form of a setscrew 54. The setscrew 54 is seated within a threaded aperture 56 on the hub 53 to secure the flange 52 and hub 53 arrangement to shank 58 of the system 12 and against the flexible member 14. The system 12 can be assembled pre- or intra-operatively. Once assembled, the system 12 is positioned in the top loading anchor members 16 and secured thereto by the threadable cap members 26, as shown in Fig. 17B, resulting in the arrangement and installation of the flexible member 14. A cross-sectional view of the system 12 and associated anchor members 16 of Figs. 17A and 17B is shown in Fig. 17C.

The materials that may be used in the flexible members 14 of the present invention can be selected from any suitable biocompatible material as known in the art. By way of example, the materials can include rigid or flexible metals, ceramic materials, carbon fiber, polymeric materials, and/or composite materials. The metals can include titanium or nickel-titanium alloy (NiTiNOL) wire, such as superelastic or shape memory NiTiNOL, for example. The polymeric materials can include, for example, hydrogels (e.g., polyacrylamides), silicone elastomers (natural or synthetic), epoxies (e.g., polyamide epoxy), urethanes, and thermoplastic materials, such as polyurethane, polyethylene (e.g., UHMWPE), polyethylene terephthalate (e.g., Sulene®), polypropylene, polyamide (e.g., Nylon), polyester, acetal, polycarbonate, thermoplastic elastomers, and the like. The composite materials may include, for example, resin impregnated graphite or aramid fibers (e.g., liquid crystal polymers such as Kevlar®), or NiTi dispersed in polyethylene terephthalate. It will be recognized that various other materials suitable for implantation of the flexible member 14 within the human body and for providing stabilization of the spine while maintaining flexibility may be used.

The above-described flexible members 14 can be manufactured using injection molding processes, or other suitable processes, as are known in the art. To that end, the proposed configurations may be injection molded using, for example, a one-step process or a multi-step process involving the material(s) of the flexible member 14. In addition, the desired flexible member 14 also may be extruded using a conventional thermoplastic extrusion process. Such process can utilize one or more extrusion heads having a die nozzle configuration to feed the materials into an extrusion die to form a well-fused combination of materials, i.e., to form the flexible member 14.

Accordingly, there is provided flexible member 14 having variable flexibility attributable to a specified configuration for use with a dynamic stabilization system 12 to provide dynamic stability to a person's spine 10. Such variability in flexibility, for example, can provide surgeons with greater options in selecting the most appropriate flexible member 14 for placement at a specific location along the spine 10, such selection being dictated by the desired bending movement of the flexible member 14 at that location.

While the invention has been illustrated by a description of various embodiments and while these embodiments have been described in considerable detail, the scope of the invention is limited only by the appended claims.

## Claims

1. A spine stabilization system comprising:
a first anchor member (16) and a second anchor member (16);
a flexible member (14) including a body (30) having a lengthwise axis (38), an outer surface (40), opposing first and second ends (32, 34) with an intermediate portion (36) extending therebetween, and an aperture (24) extending from the first end (32) to the second end (34); and
a flexible connecting member (22) passing through the aperture of the body of the flexible member (14), the connecting member (22) being secured to the first anchor member (16) and the second anchor member (16) such that the flexible member (14) is positioned between the first anchor member (16) and the second anchor member (16);
the outer surface of the body (30) of the flexible member (14) including one or more grooves (42, 42a, 42b,44) therein to provide the flexible member with a variable flexibility,
the spine stabilization system (12) being **characterized in that**:
the one or more grooves comprise a first groove (42, 42a, 42b) situated generally perpendicular to the lengthwise axis (38) of the body (30) and extending around less than the outer surface of the body (30).

2. The spine stabilization system of claim 1 wherein the first groove is further provided within the outer surface of the intermediate portion in-between opposing ends.

3. The spine stabilization system of claim 2 wherein the first groove further comprises a pair of first grooves situated in opposing relation and each extending around less than half the outer surface of the body (30), each first groove increasing in depth in a direction from opposing ends to a center of the groove to define a crescent-shaped groove wherein the intermediate portion is substantially oval-shaped when viewed in cross-section perpendicular to the lengthwise axis of the body (30).

4. The spine stabilization system of claim 1 wherein the first groove further includes a plurality of spaced-apart first grooves extending around no more than half the outer surface of the body (30) to provide the flexible member (14) with a variable flexibility.

5. The spine stabilization system of claim 1 further comprising a third groove (42, 42a, 42b, 44) situated perpendicular to the lengthwise axis (38) of the body (30) and extending around the outer surface of the body.

6. The spine stabilization system of claim 1 wherein each opposing end of the body (30) is in engagement with one of the anchor members (16).

7. The spine stabilization system of claim 1 wherein a width of the first groove is greater than half the length of the body (30); and/or wherein at least one end of the first groove is flared.

8. The spine stabilization system of claim 1 further comprising a second groove (42, 42a, 42b, 44) situated generally perpendicular to the lengthwise axis of the body (30) and extending around the outer surface of the body (30) substantially directly inbetween the ends (32, 34).

9. The spine stabilization system of claim 8 wherein a width of the second groove is greater than about one third and less than about two-thirds the full length of the body (30) such that the body substantially defines a dumbbell shape.

10. The spine stabilization system of claim 1 wherein the aperture extends lengthwise therethrough along a central axis.

11. The spine stabilization system of claim 10 wherein the lengthwise axis (38) defines a lengthwise central axis of the body (30) of the flexible member (14) and the central axis of the aperture extends lengthwise therethrough offset from the lengthwise central axis of the body (30) of the flexible member (14).

12. A spine stabilization system comprising:
a first anchor member (16) and a second anchor member (16);
a flexible member (14) including a body (30) having opposing first and second ends (32, 34) with an intermediate portion (36) extending therebetween, and an aperture (24) extending lengthwise through the body from the first end (32) to the second end (34); and
a flexible connecting member (22) passing through the aperture of the body (30) of the flexible member (14), the connecting member (22) being secured to the first anchor member (16) and the second anchor member (16) such that the flexible member (14) is positioned between the first anchor member (16) and the second anchor member (16);
the spine stabilization system (12) being **characterized in that**:
the flexible member (14) includes a taper in the flexible member to provide the flexible member (14) with a variable flexibility, wherein the taper is one of:
(a) a taper in diameter of the body (30); or
(b) a taper in diameter of the aperture extending lengthwise through the body (30).

13. The spine stabilization system of claim 12 wherein the aperture extends lengthwise therethrough offset from a central axis of the body (30) of the flexible member (14).

14. A spine stabilization system comprising:
a first anchor member (16) and a second anchor member (16);
a flexible member (14) including a body (30) having opposing first and second ends (32, 34) with an intermediate portion (36) extending therebetween, and an aperture (24) extending lengthwise through the body from the first end (32) to the second end (34); and
a flexible connecting member (22) passing through the aperture of the body of the flexible member (14), the connecting member (22) being secured to the first anchor member (16) and the second anchor member (16) such that the flexible member (14) is positioned between the first anchor member (16) and the second anchor member (16);
the spine stabilization system (12) being **characterized in that**:
the body (30) is substantially oval-shaped along its length when viewed from both ends (32, 34) to provide the flexible member (14) with a variable flexibility.

## Patentansprüche

1. Wirbelsäulenstabilisierungssystem, umfassend:
ein erstes Verankerungselement (16) und ein zweites Verankerungselement (16);
ein flexibles Element (14), das einen Körper (30), der eine Längsachse (38) aufweist, eine Außenfläche (40), gegenüberliegende erste und zweite Enden (32, 34) mit einem sich dazwischen erstreckenden Zwischenabschnitt (36) und eine Durchbrechung (24) aufweist, die sich von dem ersten Ende (32) zu dem zweiten Ende (34) erstreckt; und
ein flexibles Verbindungselement (22), das durch die Durchbrechung des Körpers des flexiblen Elements (14) verläuft, wobei das Verbindungselement (22) an dem ersten Verankerungselement (16) und dem zweiten Verankerungselement (16) so angebracht ist, dass das flexible Element (14) zwischen dem ersten Verankerungselement (16) und dem zweiten Verankerungselement (16) positioniert ist;
wobei die Außenfläche des Körpers (30) des flexiblen Elements (14) eine oder mehrere Nuten (42, 42a, 42b, 44) darin umfasst, um das flexible Element mit einer variablen Flexibilität zu versehen,
wobei das Wirbelsäulenstabilisierungssystem (12) **dadurch gekennzeichnet ist, dass**:
die eine oder mehreren Nuten eine erste Nut (42, 42a, 42b) umfassen, die allgemein rechtwinklig zu der Längsachse (38) des Körpers (30) angeordnet ist und sich um weniger als die Außenfläche des Körpers (30) erstreckt.

2. Wirbelsäulenstabilisierungssystem nach Anspruch 1, wobei die erste Nut ferner in der Außenfläche des Zwischenabschnitts zwischen gegenüberliegenden Enden vorgesehen ist.

3. Wirbelsäulenstabilisierungssystem nach Anspruch 2, wobei die erste Nut ferner ein Paar erster Nuten umfasst, die in gegenüberliegender Beziehung angeordnet sind und sich jeweils um weniger als die Hälfte der Außenfläche des Körpers (30) erstrecken, wobei jede erste Nut in der Tiefe in einer Richtung von gegenüberliegenden Enden zu einem Zentrum der Nut zunimmt, um eine halbmondartige Nut zu definieren, wobei der Zwischenabschnitt im Wesentlichen ovalförmig bei Betrachtung im Querschnitt rechtwinklig zu der Längsachse des Körpers (30) ist.

4. Wirbelsäulenstabilisierungssystem nach Anspruch 1, wobei die erste Nut ferner eine Mehrzahl beabstandeter erster Nuten umfasst, die sich um nicht mehr als die Hälfte der Außenfläche des Körpers (30) erstrecken, um das flexible Element (14) mit einer variablen Flexibilität zu versehen.

5. Wirbelsäulenstabilisierungssystem nach Anspruch 1, ferner mit einer dritten Nut (42, 42a, 42b, 44), die rechtwinklig zu der Längsachse (38) des Körpers (30) angeordnet ist und sich um die Außenfläche des Körpers erstreckt.

6. Wirbelsäulenstabilisierungssystem nach Anspruch 1, wobei jedes gegenüberliegende Ende des Körpers (30) in Eingriff mit einem der Verankerungselemente (16) steht.

7. Wirbelsäulenstabilisierungssystem nach Anspruch 1, wobei eine Breite der ersten Nut größer als eine Hälfte der Länge des Körpers (30) ist; und/oder wobei zumindest ein Ende der ersten Nut aufgeweitet ist.

8. Wirbelsäulenstabilisierungssystem nach Anspruch 1, ferner mit einer zweiten Nut (42, 42a, 42b, 44), die allgemein rechtwinklig zu der Längsachse des Körpers (30) angeordnet ist und sich um die Außenfläche des Körpers (30) im Wesentlichen direkt zwischen den Enden (32, 34) erstreckt.

9. Wirbelsäulenstabilisierungssystem nach Anspruch 8, wobei eine Breite der zweiten Nut größer als etwa ein Drittel und kleiner als etwa zwei Drittel der vollen Länge des Körpers (30) ist, so dass der Körper im Wesentlichen eine Hantelform definiert.

10. Wirbelsäulenstabilisierungssystem nach Anspruch 1, wobei die Durchbrechung sich in Längsrichtung hindurch entlang einer Zentralachse erstreckt.

11. Wirbelsäulenstabilisierungssystem nach Anspruch 10, wobei die Längsachse (38) eine Längszentralachse des Körpers (30) des flexiblen Elements (14) definiert und die Zentralachse der Durchbrechung sich längs hindurch versetzt von der Längszentralachse des Körpers (30) des flexiblen Elements (14) erstreckt.

12. Wirbelsäulenstabilisierungssystem, umfassend:
ein erstes Verankerungselement (16), ein zweites Verankerungselement (16);
ein flexibles Element (14) mit einem Körper (30), der gegenüberliegende erste und zweite Enden (32, 34) mit einem sich dazwischen erstreckenden Zwischenabschnitt (36) und eine Durchbrechung (24) aufweist, die sich längs durch den Körper von dem ersten Ende (32) zu dem zweiten Ende (34) erstreckt; und
ein flexibles Verbindungselement (22), das durch die Durchbrechung des Körpers (30) des flexiblen Elements (14) verläuft, wobei das Verbindungselement (22) an dem ersten Verankerungselement (16) und dem zweiten Verankerungselement (16) so angebracht ist, dass das flexible Element (14) zwischen dem ersten Verankerungselement (16) und dem zweiten Verankerungselement (16) positioniert ist;
wobei das Wirbelsäulenstabilisierungssystem (12) **dadurch gekennzeichnet ist, dass**:
das flexible Element (14) eine Verjüngung in dem flexiblen Element aufweist, um das flexible Element (14) mit einer variablen Flexibilität zu versehen, wobei die Verjüngung eines ist aus:
(a) eine Verjüngung des Durchmessers des Körpers (30); oder
(b) eine Verjüngung des Durchmessers der Durchbrechung, die sich in Längsrichtung durch den Körper (30) erstreckt.

13. Wirbelsäulenstabilisierungssystem nach Anspruch 12, wobei sich die Durchbrechung längs hindurch versetzt von einer Zentralachse des Körpers (30) des flexiblen Elements (14) erstreckt.

14. Wirbelsäulenstabilisierungssystem, umfassend:
ein erstes Verankerungselement (16) und ein zweites Verankerungselement (16);
ein flexibles Element (14) mit einem Körper (30), der gegenüberliegende erste und zweite Enden (32, 34) mit einem sich dazwischen erstreckenden Zwischenabschnitt (36) und eine Durchbrechung (24) aufweist, die sich längs durch den Körper von dem ersten Ende (32) zu dem zweiten Ende (34) erstreckt; und
ein flexibles Verbindungselement (22), das durch die Durchbrechung des Körpers des flexiblen Elements (14) verläuft, wobei das Verbindungselement (22) an dem ersten Verankerungselement (16) und dem zweiten Verankerungselement (16) so angebracht ist, dass das flexible Element (14) zwischen dem ersten Verankerungselement (16) und dem zweiten Verankerungselement (16) positioniert ist;
wobei das Wirbelsäulenstabilisierungssystem (12) **dadurch gekennzeichnet ist, dass**:
der Körper (30) im Wesentlichen ovalförmig entlang seiner Länge bei Betrachtung von beiden Enden (32, 34) ist, um das flexible Element (14) mit einer variablen Flexibilität zu versehen.

## Revendications

1. Système de stabilisation spinale comprenant:
un premier élément d'ancrage (16) et un second élément d'ancrage (16);
un élément flexible (14) incluant un corps (30) ayant un axe longitudinal (38), une surface extérieure (40), une première et une seconde extrémité (32, 34) opposées avec une portion intermédiaire (36) s'étendant entre elles, et une ouverture (24) s'étendant depuis la première extrémité (32) jusqu'à la seconde extrémité (34); et
un élément de connexion flexible (22) qui passe à travers l'ouverture du corps de l'élément flexible (14), l'élément de connexion (22) étant fixé sur le premier élément d'ancrage (16) et sur le second élément d'ancrage (16) de telle façon que l'élément flexible (14) est positionné entre le premier élément d'ancrage (16) et le second élément d'ancrage (16);
la surface extérieure du corps (30) de l'élément flexible (14) incluant une ou plusieurs rainures (42, 42a, 42b, 44) à l'intérieur pour conférer à l'élément flexible une flexibilité variable,
le système de stabilisation spinale (12) étant **caractérisé en ce que**:
lesdites une ou plusieurs rainures comprennent une première rainure (42, 42a, 42b) située généralement perpendiculaire à l'axe longitudinal (38) du corps (30) et s'étendant autour d'une étendue plus petite que la surface extérieure du corps (30).

2. Système de stabilisation spinale selon la revendication 1, dans lequel la première rainure est en outre prévue dans la surface extérieure de la portion intermédiaire entre les extrémités opposées.

3. Système de stabilisation spinale selon la revendication 2, dans lequel la première rainure comprend en outre une paire de premières rainures situées en relation d'opposition et s'étendant chacune autour de moins de la moitié de la surface extérieure du corps (30), chaque première rainure ayant une profondeur qui augmente dans une direction depuis des extrémités opposées jusqu'à un centre de la rainure pour définir une rainure en forme de croissant, dans lequel la portion intermédiaire est sensiblement de forme ovale lorsqu'on la voit en section transversale perpendiculaire à l'axe longitudinal du corps (30).

4. Système de stabilisation spinale selon la revendication 1, dans lequel la première rainure inclut en outre une pluralité de premières rainures espacées qui s'étendent autour d'une étendue qui n'est pas plus grande que la moitié de la surface extérieure du corps (30) pour conférer à l'élément flexible (14) une flexibilité variable.

5. Système de stabilisation spinale selon la revendication 1, comprenant en outre une troisième rainure (42, 42a, 42b, 44) située perpendiculaire à l'axe longitudinal (38) du corps (30) et s'étendant autour de la surface extérieure du corps.

6. Système de stabilisation spinale selon la revendication 1, dans lequel chaque extrémité opposée du corps (30) est en engagement avec l'un des éléments d'ancrage (16).

7. Système de stabilisation spinale selon la revendication 1, dans lequel une largeur de la première rainure est plus grande que la moitié de la longueur du corps (30); et/ou dans lequel au moins une extrémité de la première rainure est évasée.

8. Système de stabilisation spinale selon la revendication 1, comprenant en outre une seconde rainure (42, 42a, 42b, 44) située généralement perpendiculaire à l'axe longitudinal du corps (30) et s'étendant autour de la surface extérieure du corps (30) sensiblement directement entre les extrémités (32, 34).

9. Système de stabilisation spinale selon la revendication 8, dans lequel une largeur de la seconde rainure est plus grande qu'environ un tiers et plus petite qu'environ deux tiers de la longueur totale du corps (30) de telle façon que le corps définit sensiblement une forme en haltère.

10. Système de stabilisation spinale selon la revendication 1, dans lequel l'ouverture s'étend longitudinalement à travers celui-ci le long d'un axe central.

11. Système de stabilisation spinale selon la revendication 10, dans lequel l'axe longitudinal (38) définit un axe central longitudinal du corps (30) de l'élément flexible (14) et l'axe central de l'ouverture s'étend longitudinalement à travers celui-ci et en décalage depuis l'axe central longitudinal du corps (30) de l'élément flexible (14).

12. Système de stabilisation spinale comprenant:
un premier élément d'ancrage (16) et un second élément d'ancrage (16);
un élément flexible (14) incluant un corps (30) ayant une première et une seconde extrémité (32, 34) opposées avec une portion intermédiaire (36) s'étendant entre elles, et une ouverture (24) s'étendant longitudinalement à travers le corps depuis la première extrémité (32) jusqu'à la seconde extrémité (34); et
un élément de connexion flexible (22) qui passe à travers l'ouverture du corps (30) de l'élément flexible (14), l'élément de connexion (22) étant fixé au premier élément d'ancrage (16) et au second élément d'ancrage (16) de telle façon que l'élément flexible (14) est positionné entre le premier élément d'ancrage (16) et le second élément d'ancrage (16);
le système de stabilisation spinale (12) étant **caractérisé en ce que**:
l'élément flexible (14) inclut un évasement dans l'élément flexible pour conférer à l'élément flexible (14) une flexibilité variable, dans lequel l'évasement est un évasement parmi:
(a) un évasement de diamètre du corps (30); ou
(b) un évasement de diamètre de l'ouverture s'étendant longitudinalement à travers le corps (30).

13. Système de stabilisation spinale selon la revendication 12, dans lequel l'ouverture s'étend longitudinalement à travers celui-ci et en décalage depuis un axe central du corps (30) de l'élément flexible (14).

14. Système de stabilisation spinale comprenant:
un premier élément d'ancrage (16) et un second élément d'ancrage (16);
un élément flexible (14) incluant un corps (30) ayant une première et une seconde extrémité opposées (32, 34) avec une portion intermédiaire (36) s'étendant entre elles, et une ouverture (24) s'étendant longitudinalement à travers le corps depuis la première extrémité (32) jusqu'à la seconde extrémité (34); et
un élément de connexion flexible (22) qui passe à travers l'ouverture du corps de l'élément flexible (14), l'élément de connexion (22) étant fixé au premier élément d'ancrage (16) et au second élément d'ancrage (16) de telle façon que l'élément flexible (14) est positionné entre le premier élément d'ancrage (16) et le second élément d'ancrage (16);
le système de stabilisation spinale (12) étant **caractérisé en ce que**:
le corps (30) est sensiblement de forme ovale le long de sa longueur lorsqu'on le voit depuis les deux extrémités (32, 34) pour conférer à l'élément flexible (14) une flexibilité variable.
